(19) **Europäisches Patentamt**

**European Patent Office**

**Office européen des brevets**

(11) **EP 1 622 059 A1**

(12) **EUROPEAN PATENT APPLICATION**

(43) Date of publication:
**01.02.2006 Bulletin 2006/05**

(51) Int Cl.:
***G06F 19/00*** (2006.01)

(21) Application number: **05254765.0**

(22) Date of filing: **29.07.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **30.07.2004 US 592919 P**
**13.07.2005 US**

(71) Applicant: **ETHICON ENDO-SURGERY, INC.**
**Cincinnati, Ohio 45242 (US)**

(72) Inventors:
• **Shaya, Steven**
**Highlands**
**New Jersey 07732 (US)**

• **Brenner-Gati, Leona**
**Princeton Junction**
**New Jersey 08550 (US)**
• **Cronin, John E.**
**Jericho**
**Vermont 05465 (US)**
• **Drehwing Edwards, Nancy**
**Jericho**
**Vermont 05465 (US)**
• **Roth, Douglas, J.**
**Essex**
**Vermont 05452 (US)**

(74) Representative: **Tunstall, Christopher Stephen et al**
**Carpmaels & Ransford,**
**43-45 Bloomsbury Square**
**London WC1A 2RA (GB)**

(54) **A method and apparatus for measuring and controlling food intake of an individual**

(57) Provided herein is a method for monitoring dietary intake of food items. The method involves the steps of selecting a portion of food, weighing the portion, inputting the type and weight amount of the food into a computer, and obtaining the caloric content of the food by from a database. There is also provided an apparatus for monitoring dietary intake of food items. The apparatus includes a means for weighing a preselected portion of food, a computer for inputting the type and weight amount of the food therein, and a database operatively associated with the computer for obtaining the caloric content of the food.

FIG. 3C

**Description**

**CROSS REFERENCE TO RELATED APPLICATIONS**

This application claims the benefit of U.S. Provisional Application Ser. No. 60/592,919 entitled "A METHOD AND APPARATUS FOR MEASURING AND CONTROLLING FOOD INTAKE OF AN INDIVIDUAL" to Shay, filed 30 July 2004.

Field of the Invention

The present invention relates in general to methods and apparatuses for measuring caloric or other nutritional component intake of an individual.

Background of the Invention

The percentage of the world's population suffering from obesity is steadily increasing. Severely obese persons are susceptible to increased risk of heart disease, stroke, diabetes, pulmonary disease, and accidents. Because of the effect of morbid obesity to the life of the patient, methods of treating morbid obesity are being researched.

Numerous non-operative therapies for morbid obesity have been tried with virtually no permanent success. Dietary counseling, behavior modification, wiring a patient's jaws shut, and pharmacological methods have all been tried, and failed to correct the condition. Mechanical apparatuses for insertion into the body through non-surgical means, such as the use of gastric balloons to fill the stomach have also been employed in the treatment of the condition. Such devices cannot be employed over a long term, however, as they often cause severe irritation, necessitating their periodic removal and hence interruption of treatment. Thus, the medical community has evolved surgical approaches for treatment of morbid obesity. Dieting and diet pills are used repeatedly by the obese in part because these programs are generally unsuccessful and consumers relapse.

The failure of the non-operative approaches may lay in the difficulties in knowing and keeping track of accurate food consumption parameters that have a direct bearing on caloric and other nutritional component consumption, but also pertains to other nutritional components such as fat content, salt levels, etc. Accurate portion monitoring is a key variable in weight management and diabetic glycemia management programs. Real time portion feedback is a new way for dieters to take more than they need to eat, but get information and signals as they eat that their limits have been met. Once there is a measure of caloric intake, there is a need to correlate this with the dependent variables, weight and blood glucose readings, also integrating data on the other independent variables, caloric expenditure data or exercise records, drug dosing, illness, stress, and so forth. That is to say, a management system is empowered once there is access to detailed and accurate dietary data at the level of average daily intake plots, breakouts by meals, day of the week, and, if necessary, the ability to dive down to the food components of out of limits meals. The algorithms used to drive insulin pump delivery require blood glucose and would be improved by addition of caloric or carbohydrate intake as an independent parameter. This device provides timed caloric intake accurate to about 10 seconds. (The uncertainty between time of lifting a bit of food from the plate and ingesting it.) No previous device addresses this need prior to this invention. While caloric content of food is commonly utilized for rational management of weight, other nutritional components of consumed food are of value in a range of medical conditions. These include carbohydrates, fats or lipids, saturated or unsaturated fatty acids, sodium, protein, dietary fiber, sugar, calcium, iron, and various vitamins. All of these can be tracked in a way similar to calories by this invention. In the following, where calories are described as the subject of monitoring, it is understood users could be monitoring other nutritional components, multiple nutritional components or properties, or parameters that are a combination of multiple food parameters. The communication of these data can be numerical, relative to personal targets, or qualitative, as in a good/bad, healthy/unhealthy, green light/red light or similar qualitative communication.

Weight management programs utilize data entry formats (food consumption logs) for the energy input side of the balance against energy utilization. This is awkward, relies on memory, involves estimates of quantities that many people have no comprehension for, and is often subject to biased estimations. Even a crude input of all factors involved in diabetic management provides a platform for more informed patient management. The consideration of all components impacting the patient outcomes is appreciated by the patient. This data provides the professional with a picture of the lifestyle management challenges. At this time, the outcome drivers, food and exercise, are poorly measured. We are seeking ways to upgrade this glaring inadequacy. What is needed is a way to help manage dietary intake important to diabetics, overweight, underweight, geriatric, pregnant and people managing their weight and other health problems for a variety of reasons.

**Brief Summary of the Invention**

Provided herein is an apparatus for and method of monitoring dietary intake of food items. The apparatus includes a means for weighing a pre-selected portion of food, a computer for inputting the type and accepting the weighed amount of the food, further having a database operatively associated with the computer for obtaining the caloric or nutrient content of the actual amount of each food type consumed, and a display and keyboard designed to interact with the computer and database to use the apparatus.

The apparatus weighs portions and tracks either changes in weight of foods as they are consumed, or alternatively the weight of the residual foods ("leftovers") at the end of a meal.

There is also provided a method for monitoring dietary intake of food items utilizing the apparatus. The method involves the steps of selecting a portion of food, weighing the portion, inputting the type and weighed amount of the food into a computer, and obtaining the caloric content of the food from a database.

**Brief Description of the Figures**

**Figure 1** is a high-level diagram of a real-time calorie counting system **100**.

**Figures 2A** and **2B** are detailed functional block diagrams of real-time calorie counting system 100.

**Figures 3A and 3B** illustrate weight sensor **50** and weight sensor subsystem **55.**

**Figure 3C** is a top view of real-time calorie counting system **100** including the interactive display, made in accordance with the present invention.

**Figure 3D** is a cross section of the real time calorie counting system **100** shown in **Figure 3C.**

**Figure 4** illustrates a flow diagram of a method **400** for operating real-time calorie counting system **100**.

**Figure 5** illustrates a flow diagram of a method **500** for calculating the caloric content of a food item.

**Figure 6** is a method of determining calorie content of a homemade recipe.

**Detailed Description of the Invention**

The present invention is an apparatus and method for monitoring dietary intake of food items, including operational systems and algorithms to accomplish this, and business methods that employ this apparatus.

The apparatus monitors and tracks energy input or other nutritive components for a user. The user enters the identity of each kind of food being consumed using any of a variety of search methods. The identity of food can also be automatically determined by a bar code reader to read packaging product identification. The weight of a food consumed is logged into a computer by any of a variety of modes: direct entry, weighing of foodstuff portions, or by weighing of foodstuff actually consumed. The latter is accomplished in one of two ways: 1) the multiple food items that have been plated are selectable by the dieter (by touch screen on their identifiers or a dropdown menu, etc.) while that food item is being consumed and continuously weighed, allowing real-time display of calories consumed; or 2) the leftover portions of each food item is designated, removed, and that weight discounted from the portion of that food item initially ascertained. The apparatus has and a display, which displays the calories of food portions as they are added to a plate and/or while food is being consumed. These real-time readings may then be displayed along with the deviation from personal caloric intake targets for each meal and/or the whole day. Thereafter the over/under target differences may also displayed. This real time display and signaling provides dieters the information they need to eat responsibly. This device records the identity of each food, the weight consumed, the caloric content consumed, and the time of consumption for a variety of dietary analyses as well as downloading of this data for external analyses.

A method of tracking accurate caloric intake by weighing food portions, identifying the food item, and having the device automatically multiply the specific energy by the weight of food to keep records of that intake is also provided and described herein. In addition, there is provided herein a method to display the running caloric intake as food is removed from a plate spoonful by spoonful. These methods may also involve the step of displaying the deviation of the real time or portioned food intake against daily and meal targets.

**Real Time Calorie Counting System**

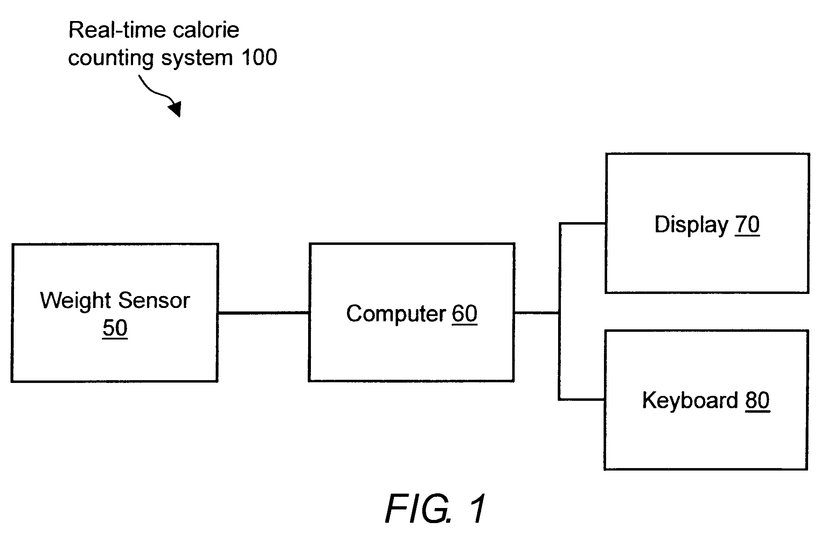

**Figure 1** is a high-level diagram of a real-time calorie counting system **100,** including a weight sensor **50,** a computer **60,** a display **70,** and an input device, such as a keyboard **80.**

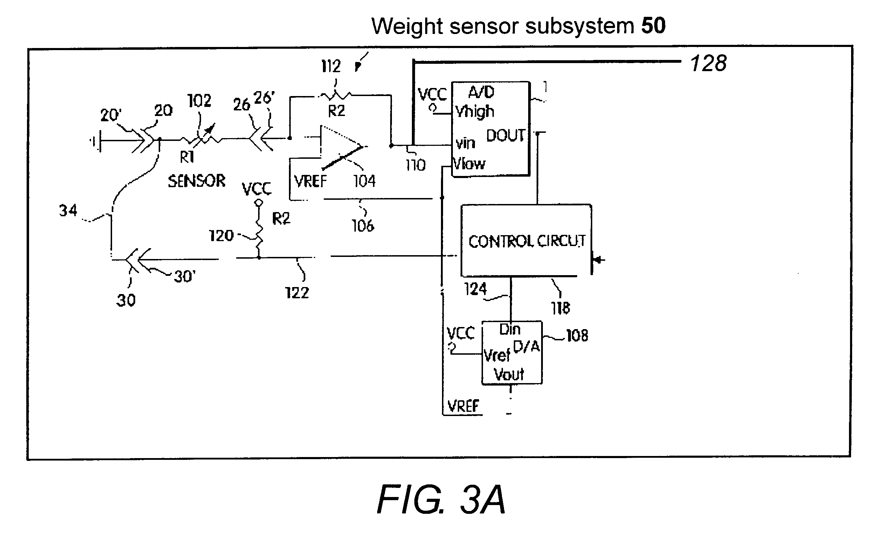

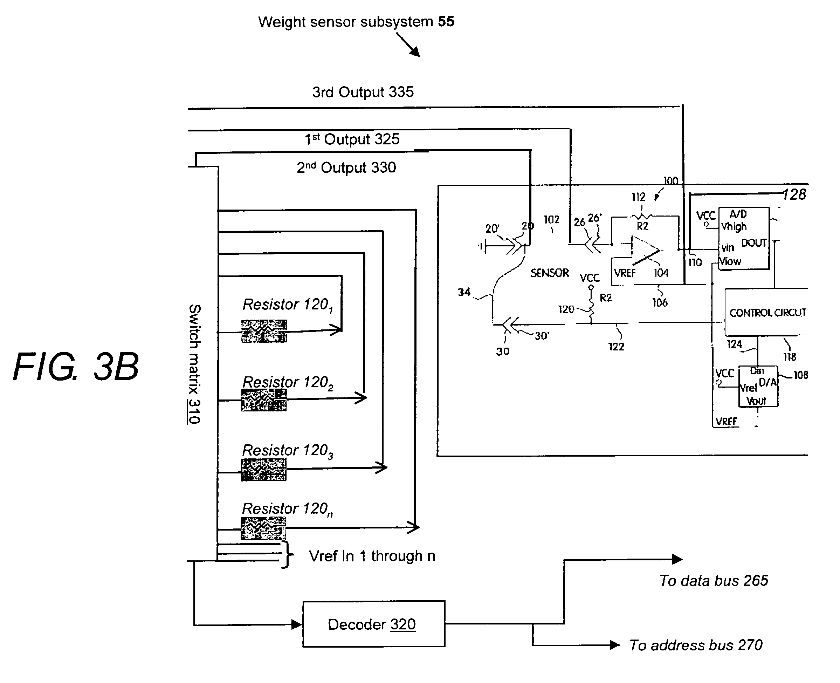

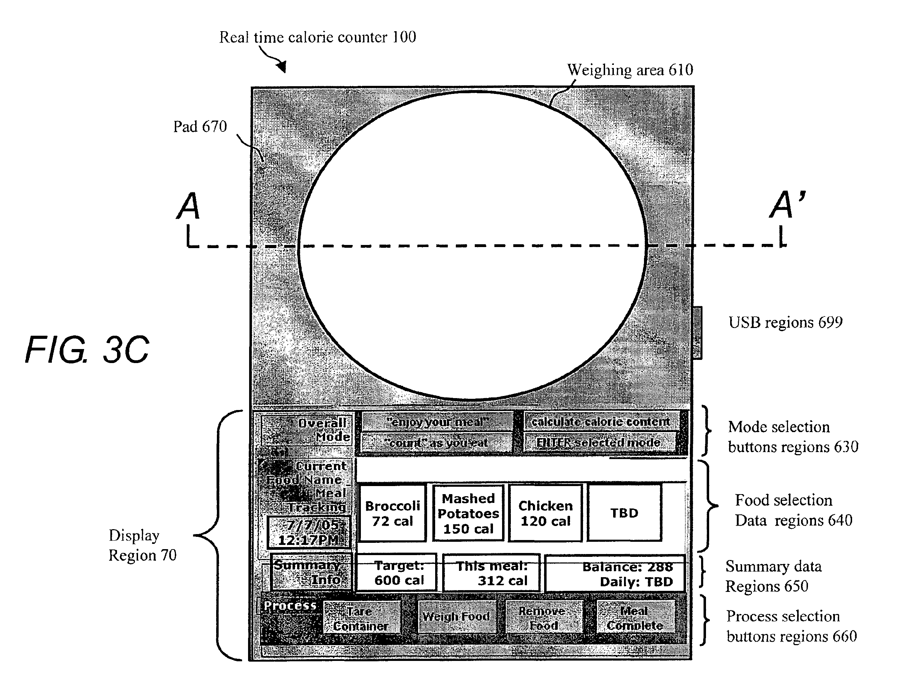

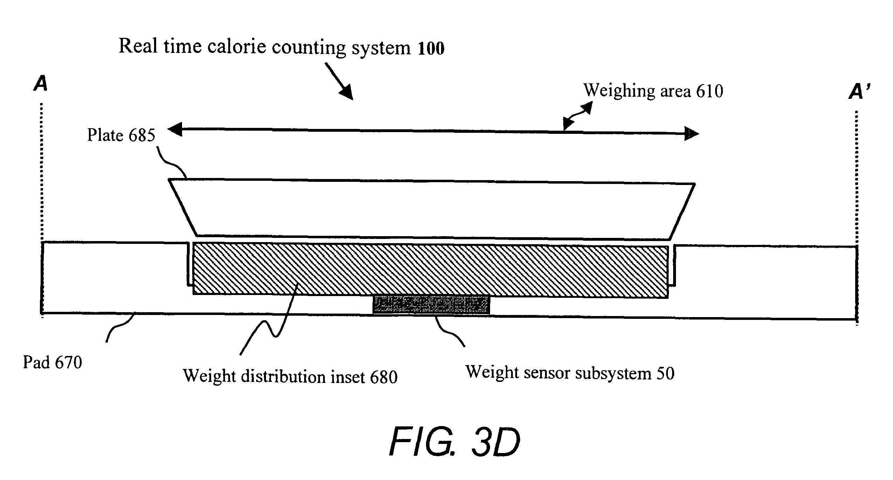

An individual can weigh food items or food containers, such as a plate, using weight sensor **50** (described in further detail in **Figure 3** below). The food items being weighed may be placed on weight sensor **50** individually or grouped together, such as an entire meal. The food items may be weighed prior to eating or while eating. Alternatively, the portions remaining after having eaten can be weighed. Weight sensor 50 may be powered by batteries or plugged in. Further detail on weight sensor **50** is found in **Figure 3** below.

Computer **60** is a computing device such as a personal computer (PC) or personal digital assistant (PDA) or a integrated computer in the overall real time calorie counting system **100** capable of executing software programs and storing data.

In operation, a user can determine the calorie content of a food item by placing the food item contained on or in a food container, such as a plate, on weight sensor **50.** The weight of the food container may be weighed prior to placing the food item on it, or the weight of the food container may be stored on computer **60.** The weight of the food item and food container is determined by weight sensor **50** and the weight measurement data is transmitted to computer **60.** Computer **60** deducts the weight of the food container to determine the weight of the food item only. A user selects or inputs the name or type of food item into keyboard **80.** Computer **60** receives the data input from keyboard **80,** calculates the total calories in food item, and stores the data along with the time and date. Computer transmits the information to display **70** for the user to see the information.

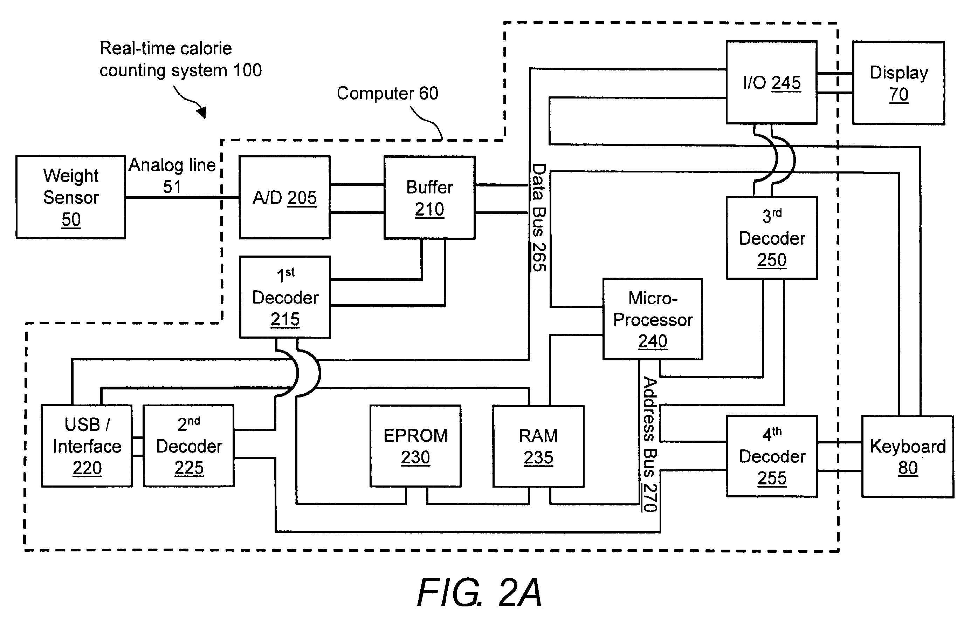

**Figure 2A** is a detailed functional block diagram of real-time calorie counting system **100**. Real-time calorie counting system **100** further includes weight sensor **50**, display **70,** a keyboard **80,** a computer **60,** which further includes an analog-to-digital (A/D) converter **205,** a buffer **210,** a decoder **215,** a universal serial bus (USB) **220,** an input-output (I/O) device **225,** an electrical programmable read-only memory (EPROM) **230,** a random access memory (RAM) device **235,** a microprocessor **240,** an I/O **245,** a decoder **250,** a decoder **255,** a data bus **265**, and an address bus **270**.

The functional blocks of real-time calorie counting system **100** are connected as shown in **Figure 2A.**

In general, microprocessor **240** controls the operation of real time calorie counting system **100** (system **100**). Software instructions programs (not shown) are stored in EPROM **230.** Data that is obtained in system **100** is stored in the RAM **235.** In general, microprocessor **240** sends address data on the address bus **270** to all devices connected to address bus **270.** Only those devices that decode their specific addresses are initialized. In general, all data goes to and from microprocessor **240** using the data bus **265.** Only those devices that are activated by the addressing of the device can send data to the microprocessor **240** and receive data from the microprocessor **240.**

Display **70** is a device, such as a CRT or LCD, which provides visual feedback to the user. Display **70** receives input from computer **60.** Display **70** is interfaced to the data bus **265** through I/O **245.** I/O **245** is any of a standard type of display drive devices that may include its own memory devices, its own decoders etc. The display **70** is addressed by address bus **270** through 3$^{rd}$ decoder **250. I/O 245** is then available to be activated and allows data through data bus **265.**

Keyboard **80** is a device, such as a keyboard, touch screen, buttons, etc., that allows a user to input data into real-time calorie counting system **100.** Keyboard **80** provides data to computer **60.** Keyboard **80** sends data to data bus **265** and hence to microprocessor **240** when the address accessing the keyboard **80** is made through 4$^{th}$ decoder **255** connected to address bus **270,** which connects to microprocessor **240.**

EPROM **230** has its own internal decoder and microprocessor **240** accesses EPROM **230** through address bus **270** and then sends or receives data from microprocessor **240** through data bus **265.**

RAM **235** has its own internal decoder and microprocessor **240** accesses RAM **235** through address bus **270** and then sends or receives data from microprocessor **240** through data bus **265.**

USB/interface **220** is an external connection to the microprocessor **240** to send or receive data to other computers or computer interfaces (not shown). USB/interface **220** sends or receives data to microprocessor **240** through data bus **265** when microprocessor **240** accesses USB/interface **220** though the 2$^{nd}$ decoder **225** when the correct address is sent on address bus **270.**

Computer **60** is capable of receiving weight data from weight sensor **50.**

Weight sensor **50** continually sends out an analog signal on analog line **51** that represents the real time weight of the system using weight sensor **50.** Analog to digital converter **205** converts the analog signal on analog line **51** to digital data using Analog to Digital converter (A/D) **205.** The digital data is continually sampled and loaded onto buffer **210** though standard means whereby the buffer **210** samples the output of A/D **205.** When the microprocessor **240** sends the correct address on address bus **270,** 1$^{st}$ decoder **215** decodes this correct address and then initializes buffer **210** to make the digital data representing the real time weight to data bus **265** to microprocessor **240.**

In operation, real-time calorie counting system **100** is first programmed with data, including, but not limited to the calorie and other nutritional component content of various food items, user health profile (e.g. age, height, and weight), exercise data and other periods of energy expenditure, and diabetic blood glucose readings. The data may be downloaded to real-time calorie counting system **100** through USB **220** to microprocessor **240.** The data is stored in RAM **235.** To determine the calorie content of a food item, a user places the item on weight sensor **50.** A user may select various modes of operation by using keyboard **80** to enter or select from a variety of options and modes, for example weigh food, "count as you eat", and "enjoy your meal." The options available and information entered may be displayed on display **70.**

When an option or mode is selected whereby real-time calorie counting system **100** requires the weight of a food item, microprocessor **240** sends address information on address bus **270** requesting to communicate with weight sensor **50** that is received by decoder **215.** Information and instructions are then able to be transferred between microprocessor **240** and weight sensor **50.** A food item is weighed by weight sensor **50,** and the weight data is converted from an analog signal to a digital signal by A/D **205.** The data is buffered by buffer **210** and received by microprocessor **240.**

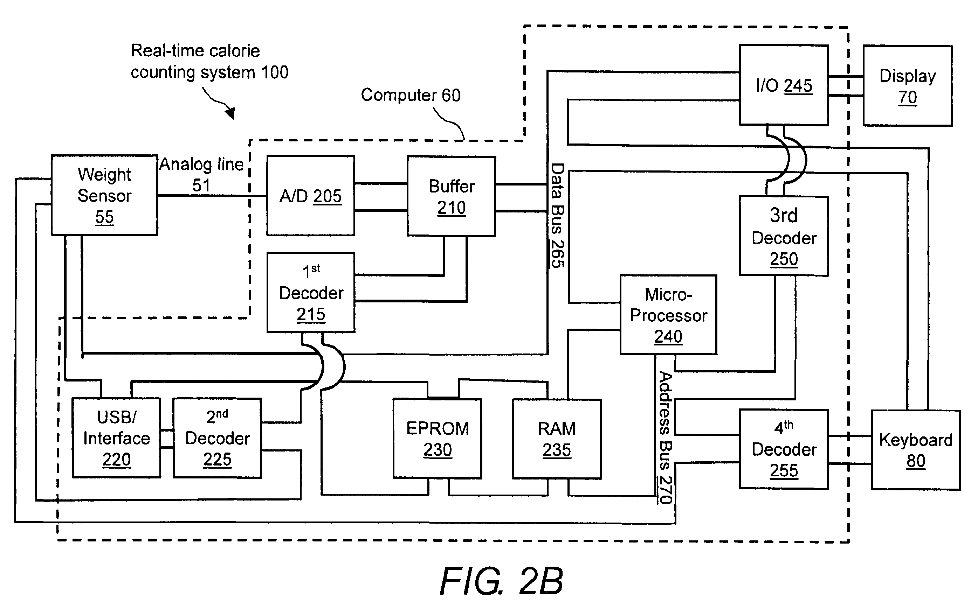

**Figure 2B** illustrates an alternate arrangement for the functional blocks of real-time calorie counting system **100,** where multiple inputs are included in weight sensor 55 and require separate decoding, address, and data lines. In the alternate arrangement, everything is identical to **Figure 2A** with the exception of added connections from data bus **265** and address bus **270** to weight sensor **55.** This design and its operation are explained in detail below in **Figure 3B.**

**Figure 3A** illustrates the design of weight sensor **50,** which is a pressure sensitive conductive ink force sensor system. Weight sensor **50** operates in a general range of 0 to 10 kg to accommodate the weight of a plate and a meal. Preferably, it has a resolution and accuracy better than 0.1 gr.

In one example, weight sensor **50** is illustrated by the Tekscan "FlexiForce" sensor system, which consists of an ultra-thin, flexible printed circuit to create a force sensor. The force sensors are constructed of at least two layers of substrate (e.g. polyester/polyimide) film. On each layer, a conductive material (silver) is applied, followed by a layer of pressure-sensitive ink. Adhesive is then used to laminate the two layers of substrate together to form the force sensor. The active sensing area is defined by the silver circle on top of the pressure-sensitive ink. Silver extends from the sensing area to the connectors at the other end of the sensor, forming the conductive leads. These sensors measure resistance, which is inversely proportional to force. The linearized force measurements are translated into weight measurements, for real-time calorie counting system 100. This design provides the needed form factor and flexibility required for the invention.

**Figure 3A,** derived from U.S. Patent Number 6,272,936 and incorporated by reference herein, shows an example of weight sensor **50,** which may be used in real-time calorie counting system **100.** All of the components of **Figure 3A** are identical to U.S. Patent Number 6,272,936 and works as described in the reference. Line **128** was added to be the analog voltage out or analog line **51** of **Figure 2A.** Weight on sensor R1 **102** of **Figure 3A** causes a corresponding analog voltage on **128** of **Figure 3A** and hence an analog voltage on analog line **51** of **Figure 2A.**

It may be possible for capacitive pressure sensors to be used that change the frequency of a tuned circuit in response to applied pressure. Examples of such sensors are M100 Miniature Planar Beam Load Cell, stainless steel low profile sensors available with ratings of 1, 2, 5, pounds, and Mini Pressure Washer (g) Pressure Sensor, only 1/8 inch tall, these stainless steel pressure sensors come in a variety of pressure ratings from 10 to 2000 grams. Model M1025, Muse Measurements 276 E. Arrow Highway, San Dimas, CA 91773. However there are likely challenges in minimizing both the cost of the device and the thickness and flexibility of the pad in using this type of sensor and thus the piezoelectric solution is preferred.

As illustrated by weight sensor subsystem **55** in **Figure 3B,** there exists a further expansion of weight sensor **50** provided through establishing a set of pressure sensitive sensors $120_1$ through $120_n$. These pressure sensitive sensors $120_1$ through $120_n$ are judiciously designed in terms of amount of conductive ink and surface are of conductive ink to optimize the weight to resistance changes. So, for example, pressure sensitive sensor $120_1$ could be a 5-10 lb weight response for a given resistance range, whereas pressure sensitive sensor $120_2$ could be designed for a 2-5 lb weight change for a given resistance range, and pressure sensitive sensor $120_3$ could be 1-2 lbs. for a given resistance change and pressure sensitive sensor $120_4$ could be designed for 0 to 1 lb weight change for a given resistance change. Resistors $120_n$ are networked via switch matrix **310** to provide varied arrangements of resistance in series and/or parallel as required for varying load capacity. Because the load of weight sensor subsystem **55** real time calorie counting system **100** will vary significantly with the types and amounts of foods added, weight sensor subsystem **55** needs to accommodate this range of load dynamically and without loss of functionality.

The switch matrix **310** of **Figure 3B** is designed to switch any of the pressure sensitive sensors $120_1$ through $120_n$ into the circuit at points **20** and **26** through output connection 1st Output **325** and 2nd Output **330** respectively. The switch matrix **310** is controlled via decoder **320** through addressing it from address bus **270** of **Figure 2B** and controlled in terms of how the pressure sensitive sensors $120_1$ through $120_n$ are switched into the circuit when from data on data bus **265** of Figure **2B.**

"Vref In" 1 through n of **Figure 3B** are switched to the VREF **106** of the circuit through connection of 3rd Output **335.** Reference voltages are needed to be switched as the pressure sensitive sensors $120_1$ through $120_n$ are switched so that the operational amplifier **104** is optimized. Thus the dynamic range can be controlled as the **Vref In 1 through n** voltages, and the pressure sensitive sensors $120_1$ through $120_n$ are changed and adapted in the circuit.

In operation, food is placed on real time calorie counting system **100** and activates weight sensor subsystem **55.** The lowest range, smallest weight, highest sensitivity pressure sensitive sensors $120_1$ through $120_n$ is the default setting before any weight is detected. As the weight is applied to the pressure sensor load via the lowest range sensor, the sensor is applied across **20** and **26** of the circuit to produce an analog output **128 of Figure 3B** to analog line **51** of **Figure 2B** to A/D converter **205** to buffer **210** to microprocessor **240** via data bus **265.**

Algorithms in EPROM **230** are used by microprocessor **240** to determine that the range detector is at the maximum range of the pressure sensor, and whether to switch to the next less sensitive sensor. If a switch between sensors and references voltages is required because the sensors are at the top or bottom of its range, the value of that sensor is stored in RAM **235.** The microprocessor **240** then switches to the higher or lower pressure sensor by addressing the decoder **320,** which then activates the switch matrix **310** through address bus **270,** and then sends the data through the data bus **265** to the decoder **320.** This action prompts switch matrix **310** to switch in the new pressure sensor or pressure sensors $120_1$ through $120_n$ to adjust resistance and voltage references VREF In 1-n, output **325** and 2nd output **330,** and thus accurately measure the load being sensed by weight sensor subsystem **55.**

By using the knowledge and data stored about what the last measurement was, in conjunction with the new sensor data in its range, the new total weight can be obtained with high sensitivity. In this manner the design of weight sensor subsystem **55** is able to accommodate and measure small loads, larger loads, and small or large changes in loads.

**Figure 3C** illustrates a top down view of real time calorie counting system **100,** made in accordance with the present invention. Real time calorie counting system **100** includes weighing area **610,** display region **70** that is of the touch screen type that further having mode selection buttons regions **630,** food selection data regions **640,** summary data regions **650** and process selection buttons regions **660.**
Weighing area **610** is the area where the plate is positioned.

Mode selection buttons regions **630** allow the user to select "Overall Mode", "Enjoy your meal", "calculate calorie content", ""count" as you eat" and "ENTER selected mode". In these regions, the user can touch the display regions and provide input data to the system.

Process selection buttons regions **660** provide operation of real time calorie counting system **100** include "Tare container", "Weigh Food", Remove Food" and "Meal Complete". In these regions, the user can touch the display regions and provide input data to the system.

Food selection Data region **640** provide a means for selecting food types. Optionally, the food selection screen may include the specific nutritive content of the food, for example, if calories are being monitored, "Broccoli 7.2 cal/gr", Mashed Potatoes 15.0 Cal/gr", Chicken 12.0 Cal/gr and "More". ". In these regions, the user can touch the display regions and provide input data to the system. The "More" button allows the user to select other choices.

The summary data region **650** shows resulting data "Target: 600 CAL", "This meal: 312 cal", balance 228, Daily TBD". In this region the resultant data "target : 600 cal" is calculated from the target calories per day inputted by the user , where as the "This meal: 312" and "balance: 228" , Daily: TBD" is calculated from the calories determined from the weight of the present meal (312 calories) minus the inputted target 600 calories leaving 228 calories

The other data in display region **70** is status information data such as "Current Food name", Meal tracking", "date (7/7/05) and time (12:17pm)" and "summary info".

In operation, for example, mode selection button regions **630** and process selection regions **660** permit selection of food names for multiple meal selections.

In an alternate embodiment, real time calorie counting system **100** may be linked to a separate PDA device (not shown) via USB region **699** that relates to USB **220** of **Figure 2A** and 2B. The real time calorie counting system **100** may link to other computers or storage devices to allow external control of the real time calorie counter **100** of **Figure 1** or simply to load in the food type, weight, or calorie database.

Cross section A-A' is further described in **Figure** 3D and is a cross section through the real time calorie counter **100** of **Figure 3C.**

**Figure 3D** shows a cross section A-A' of real time calorie counting system **100,** which includes weighing area **610** that describes the identical weighing area of **610** of **Figure 3C,** pad **670,** a weight distribution inset **680,** a plate **685,** and weight sensor **50.** In general, pad 670 is water-resistant and easy to clean, flexible material such as foam rubber. Weight distribution inset **680** is made of a hard semi-rigid plastic. Weight sensor **50** is the physical representation of the weight sensor **50** of **Figure 1,** and **Figure 2A** and also weight sensor **55** of **Figure 2B.**

Pad **670** is a flexible material, such as foam rubber, that provides support yet is still compact and can be rolled into a cylinder for use of storing in a small space. Weight distribution inset **680** provides a degree of rigidity and support to weighing area **610,** which is needed to help more evenly distribute the force and thus the measured weight across plate **685.** Weight sensor **50** is located underneath the center of weight distribution inset **680** to measure at the most central point under real time calorie monitoring system **100.**

The real time calorie counting system **100** may operate independently of an external computer at the time of food consumption. In this case, the real time calorie counting system **100** has memory of the food identification (names entered by a keyboard or keyboard simulation) and the final weights of foods consumed. Memory is adequate for many days of such data. The information is downloaded when convenient for management by a computer based software program with internet connectivity. A real time calorie counting system **100** having full food table capabilities and computer power is also easily envisioned.

Real time calorie counting system **100** can be embodied in a variety of formats. This reflects the possibility of separating a) weighing functions, b) food identity inputting, c) lookup of stored specific energy of the identified food, d) storage of meal data, and e) downloading and up loading of data to internet sites.

The embodiments can be of a variety of system designs.

i. One Piece: a self-contained device format has weighing capability, and processing functions. It operates independently.

ii. Separate Weighing Modules: A weighing module can be used portably storing food identities and weighing data. It would be connected to a processing module once a day or once every few days to down load food portion weights and food identities as a function of data and time. The processing module could service any combination of multiple users and multiple weighing modules when the weighing modules are connected to the processing module.

iii. Multiple Weighing Modules with Single Processing Module: A processing module could service multiple weighing modules simultaneously. For example, a family can have a meal together with each member of the family having their own weighing module. The processing module can inform each weighing module of the real time consumption level and target gaps.

Conventional plates may be used even when multiple foods are on the single plate or a novel multi-compartment plate may output weight for three or four demarked food areas.

In another example the pad itself can be a washable and segmented plate that has sections for different foods. Each section has its own weighing mechanism regions on pressure sensors. The design of weight sensor **50** in this case is modified to manage separate regions of the washable, segmented plate and report specifically on food weight, calorie content, and consumption per each segment.

Modules can be connected by USB/interface connection **220** of **Figure 2A** and **2B** or wirelessly connected (not shown). Microprocessors **240** of **Figure 2A** and **2B** can analyze individual dietary information and analyze family dietary patterns, as well. The latter exemplified by a question such as "what foods are associated with the family exceeding their calorie consumption targets?"

**Method of Operation of Real Time Calorie Counting System**

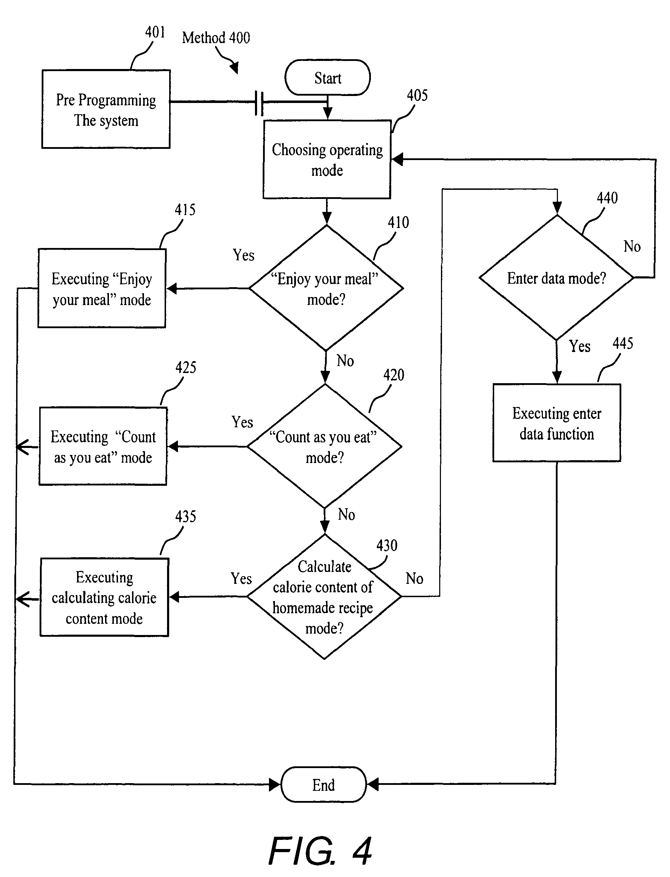

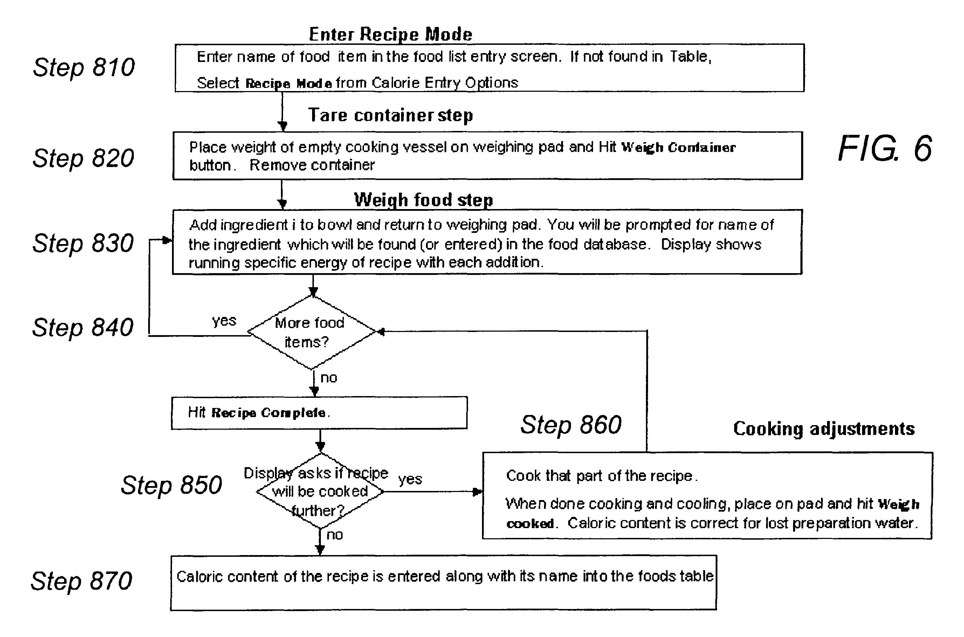

**Figure 4** illustrates a flow diagram of a method **400** for operating real-time calorie counting system **100.** These functions are included as possible PDA functions of real-time calorie counting system **100,** and shown in **Figure 6A** below.

*Step **401:** Pre Programming The System*

In this step, the system is pre-loaded with its operating software in EPROM **230.** Also, data tables are programmed into EPROM **230** that relate food type to weight to calories. See example **Table 1** below of a possible data structure to be programmed into EPROM **230.**

Table 1. Exemplary data structure for food and nutritive content table

| Food Item | Specific Calories per ounce | Typical Serving Size | Calories for typical serving size | Additional columns of specific nutritive composition |
|---|---|---|---|---|
| Chicken breast, meat & skin, roasted | 55 | 3 ounces | 165 | |
| Taco Supreme (Taco Bell) | 65 | 4 ounces | 260 | |
| Broccoli | 3.75 | 4 ounces | 15 | |
| Bread, Garlic | 120 | 1 oz slice | 120 | |
| Coffee cake | 150 | 2 oz slice | 300 | |

Also in this step, the user is also prompted for data to store in EPROM **230** such as target calories per day, per meal, and optionally health data such as beginning weight, target weight, metabolism information, etc. Software includes an interface to allow upgrading the database, addition of user specified food choices, and editing the database.

*Step **405:** Choosing operating mode*

In this step, computer **60** executes software in EPROM **230** that displays a message on display **70** prompting a user to select an operating mode. To calculate the energy of the food intake, the user selects a weighing mode from "Enjoy your meal" or "Count as you eat" calculation options.
The user is prompted to "Tare weight the container or plate" where the user places the container on pad region and enable "weigh container" function;
The method **400** proceeds to step **410** where the user is first prompted with the "Enjoy your meal" mode.

*Step **410:** Enjoy your meal mode*

In this decision step, using keyboard **80,** a user chooses whether to enter this operating mode. If the user selects this operating mode, method **400** proceeds to step **415.** If the user does not select this operating mode, method **400** proceeds to step **420.**

*Step **415:** Executing enjoy your meal function*

In this step, software stored on EPROM **230** is executed allowing a user to determine the caloric content of a food item, or group of food items, using real-time calorie counting system **100.**
The "enjoy your meal mode" programmed into EPROM **230** includes the steps of:

(1) <u>Weigh food</u> - Enable weight sensor **50.** In this step the total weight is measured, the tare of the container is subtracted to allow the system to calculate the real time exact measurement of the food added.

(2) <u>Identify food</u> - The user is prompted to enter the name of the food item from the list of prestored food types in the data stored in EPROM **230.** The user picks from the list to select from a pre-defined list. The user selects from that list or enters a new food profile. Alternatively, a bar code reader can be coupled to the EPROM **230** so that a food package can be scanned to automatically bring up the identified food;

(3) Add food, the user adds the food to the plate

(4) Calculate the calories - as the food is added, the weight is obtained and converted to the calories by simply looking up the food type selected in the data table in EPROM **230** of **Figure 2A,** using the identified weight and calorie data to determine the ratio and using this ratio to multiple by the weight found to calculate the total real time calories. Once the food type is loaded on the plate, the total calories are stored.
<u>Example:</u> The user selects "Chicken breast, meat & skin, roasted" from the list. The identified weight via weight sensor subsystem **55** is 5.5 ounces. The calories are calculated by looking up the calorie content in data tables contained in EPROM **230** and multiplying that factor by the weight of the food item as shown below:

$$5.5 \text{ ounces of chicken} * (165 \text{ calories}/3 \text{ ounce serving}) = 302.5 \text{ calories}$$

Display **70** will then show as one of the selected and loaded foods, "Chicken - 302.5 cal." The user may now select a different food type, and then start adding food to the plate and the real time calorie counting system **100** of **Figure 1** looks up the new food type in the data table stored in EPROM **230** of **Figure 2A** or **Figure 2B** and uses the new added weight and the ratio to calculate the added calories.

(5) Understanding the <u>portion size</u> where the Display **70** shows calories for all individual food items and total calories on plate. Target calories for this meal and the overall day can be shown as well;

(6) <u>Adjust portion sizes -</u> During the meal, the user may remove a food from the plate and adjust the portion size using the real time calorie counter **100** of **Figure 1** to recalculate the total calories.

(7) Store meal data - When completed with meal, enter "meal completed" to store information and move to ready mode.

Note that as an alternate approach, the name and amounts of food eaten during the day may be entered therein for later lookup of the caloric content. This type of device would then be used to only store the name and weights consumed for full calculations done later when connected to another computing unit containing the food items lookup tables. As memory capacity of portable equipment increases this becomes less necessary. Method **400** ends.

*Step **420:** Count as you eat mode?*

In this decision step, using keyboard **80,** a user chooses whether to enter this operating mode. If the user selects this operating mode, method **400** proceeds to step **425.** If the user does not select this operating mode, method **400** proceeds to step **430.**

*Step **425**: Executing count as you eat function*

In this step, software stored on EPROM **230** is executed allowing a user to determine the calories of a meal as they eat using real-time calorie counting system **100.** In the "count as you eat" mode calculation, the weight is based on assigning the currently collected weight difference to the current food item selected. To input the identity of the food in the "count as you eat" mode, the software requests the identity of food. The user begins by selecting the food type in food data selection region **640** of **Figure 3C.** The user may select the food and weigh it, subsequently the software will calculate and show the caloric content using the same process as *Step **415*** above.
The user selects the food type, eats as much as desired, selects a new food type, and eats that food, and so on. The weight change since the last food type selection is assumed to be added weight consumed of that food, added to a running sum of that food's intake; caloric intake is the sum over all foods of the running sum of each food consumed weight times its specific energy. Method **400** ends.

*Step **430**: Calculate caloric content of homemade recipe mode?*

In this decision step, using keyboard **80,** a user chooses whether to enter this operating mode. If the user selects this operating mode, method **400** proceeds to step **435.** If the user does not select this operating mode, method **400** proceeds to step **440.**

*Step **435**: Executing calculate caloric content of homemade recipe function*

In this step, software stored on EPROM **230** is executed allowing a user to determine the calories of a home cooked food item using real-time calorie counting system **100.** This method is described in detail in **Figure 6.** Method **400** ends by proceeding to Method **800.**

*Step **440**: Enter data mode?*

In this decision step, using keyboard **80,** a user chooses whether to enter this operating mode. If the user selects this operating mode, method **400** proceeds to step **445.** If the user does not select this operating mode, method **400** proceeds to step **405.**

*Step **445**: Executing enter data function*

In this step, software stored on EPROM **230** is executed allowing a user to enter names, calorie information, and other data of food items into real-time calorie counting system **100** for storage on RAM **235.** Method **400** ends.

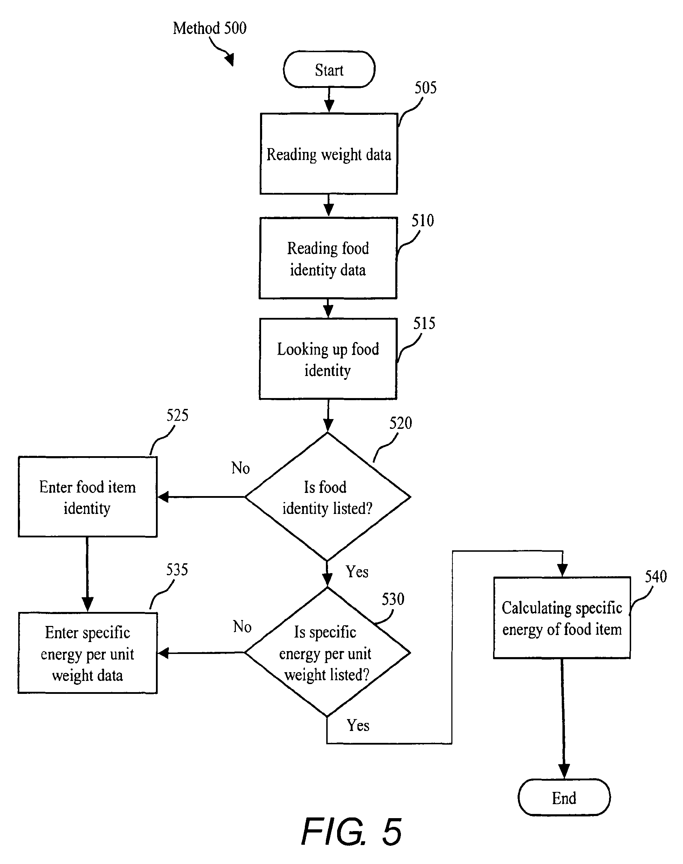

**Figure 5** illustrates a flow diagram of a method **500** for calculating the caloric content of a food item and includes the steps of:

*Step **505**: Reading weight data*

In this step, microprocessor **240** reads weight data from a memory device, such as RAM **235.** Weight data may be of a food item just weighed by weight sensor **50,** or it may be data of a previously weighed or entered food item stored in RAM **235.** Method **500** proceeds to step **510.**

*Step **510**: Reading food item data*

In this step, microprocessor **240** reads food item data from a memory device, such as RAM **235.** User enters or selects food item with keyboard **80** based on information displayed on display **70.** Food item selection is stored in RAM **235.** Method **500** proceeds to step **510.**

*Step **515**: Looking up food item*

In this step, microprocessor **240** reads database stored in RAM **235,** searching for food item selected in step **510.** Food identifiers and their specific energy are contained in the database, which may be provided with the real time calorie counting system **100** upon purchase, and further the content of RAM **235** may be updated over the Internet (or directly by the user in *Step **525*** below). Users and food manufacturers may upload new food profiles for the database at the Internet site where these may be downloaded as timed updates, e.g., "new items contributed since last update done March 14, 2005."

Calories for conventional food units (e.g., rye bread ...one slice...60 calories) may be entered or looked up. If entered, the value can be stored by the user in RAM **235** for future selection. The database information includes, at a minimum, caloric content listings (e.g., rye bread.........5.4 kcal/gr) can further provide addition dietary information, such as fat content per unit weight, sodium content, etc. When the caloric content is known, calculation of energy intake requires measuring the weight of that food consumed.

The database in RAM **235** is arranged for convenient hierarchical categorical or alphabetic selection by the user or rapid matching to input information. Method **500** proceeds to step **520**.

*Step **520**: Food item listed?*

In this decision step, microprocessor determines if food item selected in step **510** is listed in database stored in RAM **235.** If food item is not found in the database, method **500** proceeds to step **525.** If food item is found in the database, method **500** proceeds to step **530.**

*Step **525**: Enter food item*

In this step, a user is prompted by display **70** to enter the name of food item. Information is entered using keyboard **80.** When a new food is entered, a caloric content calculation page allows filling in blanks to determine the caloric content of a food using any available labeling information. New food items may be added to the database by the user via multiple sources, including but not limited to food nutrition label information, a supported recipe function, or a mail in calorimetric service. If the calories per gram is on the label that may be entered and the calculator immediately concludes with the result displayed. If calories per portion field are filled in, fields for number of portions per container and weight of container are highlighted. Method **500** proceeds to step **535.**

*Step 530: Specific energy per unit weight listed?*

In this decision step, microprocessor determines if the specific energy per unit weight of food item is listed in database stored in RAM **235.** If specific energy data is not found in the database, method **500** proceeds to step **535.** If specific energy data is found in the database, method **500** proceeds to step **540.**

*Step **535**: Enter specific energy per unit weight data*

In this decision step, a user is prompted by display **70** to enter the specific energy per unit weight of food item. The result display prompts whether the new food information should be added to the food database and in any case, the specific energy (caloric content) is displayed on the display. The display contains a section that supports entering the calories for a portion of food, weighing that portion and entering the resulting caloric content for the database. Information is entered using keyboard **80.** Method **500** proceeds to step **540.**

*Step **540**: Calculating specific energy of food item*

In this step, microprocessor **240** multiplies the specific energy per unit weight of food item by the total weight of food item to determine the total caloric content of food item. Results are stored in RAM **235.** Method **500** ends.

**Figure 6** illustrates a method of calculating caloric content (specific energy) of a homemade recipe, and includes the steps of: Step 810: Enter recipe mode, Step 820: tare container, Step 830: weigh food, Step 840: Are there more food items? Step 850: Does the recipe require further cooking? Step 860: Calculating cooking adjustments and Step 870: Entering recipe content into foods table.

When preparing food, the dish may be made from ingredients with caloric content known by the computer. Then the component fractions may be estimated or measured while the ingredients are added to the preparation bowl which is being weighed by the device. The device may then properly calculate the caloric content of the resultant mixture and final (cooked or otherwise processed) foodstuff. The final value for the prepared food may be stored within the database for future lookup functions.

The specific energy (SE) of a home-made food item, whether cooked, mixed, or constructed is entered with the aid of real time calorie. counter 100 of Figure 1. The user cooking food to determine the final calorie count would use the following method listed in **Figure 6.**

For items put together without enough cooking to lose water, the algorithm determines the weight averaged energy content for the final food product by weighing each component minus its container (the measuring spoon or cup or a tray or wrap) and entering the energy content of the ingredient from the food list. Any water added must be included as a weighed component.

$$SE = Sum\ (WiEi)/\ Sum\ (Wi)$$

If the item is cooked, the specific energy is corrected for the lost moisture of the cooking process. Any loss of energy by the burning of ingredients is minor and ignored.
$$SEc = Sum\ (WiEi)/\ Wc$$

Where SEc, the cooked energy content can also equivalently be expressed as uncooked SE x (Sum (Wi)/Wc), where Wc is the cooked weight.
The program stores the food identities for the meal, the calories of each food consumed, and the calories for the meal. This data is available for transfer to a weight management program that compares the energy intake and the energy utilization over time and identifies opportunities for particular food substitutions, showing how many calories per month would be saved by the recommended substitutions.
The advantages over prior art of the above described device and method are numerous, including: (i) no current food scales keep track of calories consumed for multiple food on a real-time basis; (ii) no current food scales provide a way to keep track of different foods all on the same plate; (iii) no current food scales correct for uneaten portions; (iv) no calorie counting system takes the weight of a food from a sensor and calculates the weight of food consumed and multiplies this by the caloric content of the food; (v) no current food databases display calories per unit weight to support weighing of food. There are no inventions addressing the need to determine the caloric content of a food.

**Business Methods for Real Time Calorie Counting**

In addition a method of doing business is provided herein. Consumers, manufacturers, or restaurateurs may weigh a new food item into a capsule and send it to a service company who will return the caloric content for that item. To determine the caloric content of any new food item, gross energy content by adiabatic bomb calorimetry is typically the industry standard. The total potential energy of foods and food components is determined by burning the food sample in a steel bomb calorimeter under elevated oxygen pressure. An initially weighed sample is sent in, dried to eliminate water, crumbled for fast combustion, burned (typically in an oxygen atmosphere), and the heat released into all combustion products and surrounding materials measured by calibration of the surrounding temperature rise and correcting for ignition thermal input. The kilocalorie (1,000 calories) is the unit commonly used in expressing energy values of a defined quantity of foods.
A simple business method in support of this process consists of the following steps:

(1) User (customer, restaurant owner, etc.) identifying a new food that requires a calorie per weight measurement;

(2) Finding a service provider of bomb calorimetry for meal planning;

(3) Sending food sample to service provider for analysis;

(4) Receiving information back from service provider; and

(5) Paying service provider for information received from analysis.

Restaurants may list the caloric content (kcal/gr) of their rapidly changing offerings in addition to the less specific kcal/portion, which is inaccurate if your portion is larger than the one assumed or if you partake of less than the so-called full portion. The device and disposable samplers are sold to restaurants so that the caloric content of meals may be provided, where required by law, while retaining the menu flexibility characteristic of a fine dining establishment. With increasing popularity of the device, restaurants and food providers may provide ID tags with their food so that the food identity may be entered by bar-scan code, RFID tag technology, or other similar product identity coding system permitting fast table lookup, encoding of relevant information, or fast downloading of the caloric content.
Current weight management programs may monitor caloric expenditure using the devices and methods mentioned above. Programs could employ patient input of food consumption. The patient input is notoriously inaccurate if not biased as to portion size. This new method replaces this input mode with measured weight of food consumed for far more accurate tracking of calories consumed. No current system provides accurate timing of caloric intake. In the count as you eat mode, the meter provides accurate timing of caloric intake. This could be used as an input for an appropriate insulin pump algorithm. The above described device and method allows users to control food portions by displaying in real time the calories plated or consumed so that a dieter may take a smaller portion or stop eating to limit caloric intake near to or below desired targets. The device may drive the delivery of insulin from a pump with signals, short of a continuous glucose sensor, from the above device based upon caloric intake.

Added Embodiments

In alternate embodiments that can be designed by one skilled in the art, data collected by real time calorie counter system **100** can be integrated with other weight management data for further analysis and user benefit. Examples of other weight management data include duration of periods of exercise, resultant calorie expenditure, markers of fat-burning metabolism, subject weight, and expected/calculated relationships among these.

For diabetic patients, a modification of real time calorie counter system **100** integrates data acquisition related to food intake such as calorie or carbohydrate intake data with an instrument that records blood glucose (BG) readings either by chemical analysis of blood samples or indirect readings that correlate with BG. Exercise history and plans can also be data incorporated for development of personalized insulin adjustment programs. Various readouts of food consumption history as well as current BG would advise the diabetic patient on adjustments to insulin dosage or pump delivery to achieve better management of glycemia. Algorithms to adjust insulin based on food intake data exist. An example of such a plan is adjusting 1U of insulin for every extra 15 gr or carbohydrates consumed (http://www.uchsc.edu/misc/diabetes/udchap21.html, table 3). Recording insulin administration and excecise further enhances the relationships that can be analyzed based on the stored data of the system. The data can be used to determine and individuals BG sensitivity to insulin and food intake to develop personalized algorithms aimed at better control of BG within normal ranges. These functions, such as analyzing blood glucose, currently exist in off the shelf products and could be integrated into a system design for the real time calorie counter system **100.** For example, using a simple time and date stamp feature in computer **60,** periods of glucose imbalance or weight gain may be identified and correlated with the meals and food categories involved in these periods of time. This information can be provided to the user or to a medical professional for assistance. The data on meal composition and caloric intake patterns provided by real time calorie counter system **100** may be uploaded to the real time calorie counter **100** of **Figure 1** to a centralized processing location (not shown) for advanced analyses, or communication in summary form to a participating physician or health care professional. The data may be downloaded, merged with other sensor information, or used in conjunction with symptom logs for support of disease management or wellness program functions. These may be used to make a range of personalized suggestions to improve desired outcomes based on the specific records of intake, exercise, drug dosage, and results recorded for the subject.

The real time calorie counter system **100 of Figure 1** and associated methods of use offer numerous consumer benefits. Users can view the caloric content of food eaten taking actual portions into consideration. Often consumers are making wise food choices, but need feedback on the portions they are consuming. As an example, should a user want to add a pat of butter to a food, within the context of the real time calorie counter system **100,** the user selects "butter" as a new food, adds butter to the plate, and reads the calories of the amount of butter added via display **70.** The consequence of the actual portion size is immediately displayed for viewing. This is the only way that a user may discover the standard portion is much less than what they are used to consuming. A key advantage of the real time calorie counter system **100** is that it replaces misconceptions and fabrications with hard data.

While the present invention has been illustrated by description of several embodiments, it is not the intention of the applicant to restrict or limit the spirit and scope of the appended claims to such detail. Numerous variations, changes, and substitutions will occur to those skilled in the art without departing from the scope of the invention. Moreover, the structure of each element associated with the present invention can be alternatively described as a means for providing the function performed by the element. Accordingly, it is intended that the invention be limited only by the spirit and scope of the appended claims.

**Claims**

1. A method for monitoring dietary caloric intake of food items, said method comprising:

    a. selecting a portion of food;
    b. weighing said portion and importing the value into a computer;

**EP 1 622 059 A1**

c. inputting the type of said food into a computer; and

d. obtaining the caloric content of said food by from a database.

e. Using the computer to calculate the caloric intake from the inputs.

2. The method of claim 1 further comprising the step of displaying and storing said caloric intake on said computer.

3. An apparatus for monitoring dietary caloric intake of food items, said apparatus comprising:

a. means for weighing a preselected portion of food;

b. a computer for inputting the type and weight amount of said food therein; and

c. a database operatively associated with said computer for obtaining the caloric content of said food.

4. The apparatus of claim 3 further comprising a means for displaying and storing said caloric content on said computer.

5. The method of claim 1 further comprising a step to correct portions size by determining an amount left unconsumed.

6. The apparatus of claim 3 further including a display to show an amount of food consumed in real time.

7. The apparatus of claim 3 further including a bar code reader for inputting the type and weight amount of said food therein.

Real-time calorie counting system 100

Display 70

Keyboard 80

Computer 60

Weight Sensor 50

FIG. 1

FIG. 2A

Real-time calorie counting system 100

Computer 60

Display 70

Keyboard 80

I/O 245

3rd Decoder 250

4th Decoder 255

Micro-Processor 240

Address Bus 270

Data Bus 265

RAM 235

EPROM 230

Buffer 210

A/D 205

1st Decoder 215

2nd Decoder 225

USB/Interface 220

Analog line 51

Weight Sensor 55

*FIG. 2B*



Weight sensor subsystem **50**

*FIG. 3A*

EP 1 622 059 A1

FIG. 3B

Weight sensor subsystem **55**

3rd Output 335

1st Output 325

2nd Output 330

128

A/D
VCC Vhigh
DOUT
Vin
Vlow
100
110

CONTROL CIRCUIT
118
108
124
VCC Din D/A
Vref Vout
VREF

112 R2
104
106
VREF
26 26'
SENSOR VCC R2
120'
122
102
20 20'
30'
30
34

Resistor 120₁
Resistor 120₂
Resistor 120₃
Resistor 120ₙ

Vref In 1 through n

Switch matrix 310

Decoder 320

To data bus 265

To address bus 270

Real time calorie counter 100

Weighing area 610

Pad 670

A

A'

USB regions 699

# FIG. 3C

| Overall Mode | "enjoy your meal" | calculate calorie content | Mode selection buttons regions 630 |
| | "count" as you eat | ENTER selected mode | |

Current Food Name
Meal Tracking
7/7/05
12:17PM

| Broccoli 72 cal | Mashed Potatoes 150 cal | Chicken 120 cal | TBD |

Food selection Data regions 640

| Summary Info | Target: 600 cal | This meal: 312 cal | Balance: 288 Daily: TBD |

Summary data Regions 650

| Process | Tare Container | Weigh Food | Remove Food | Meal Complete |

Process selection buttons regions 660

Display Region 70

EP 1 622 059 A1

Real time calorie counting system 100

Weighing area 610

Plate 685

Weight sensor subsystem 50

Weight distribution inset 680

Pad 670

FIG. 3D

FIG. 4

Method 500

```
                    ┌─────────┐
                    │  Start  │
                    └────┬────┘
                         │
                         ▼                    505
              ┌────────────────────┐
              │ Reading weight data│
              └─────────┬──────────┘
                        │
                        ▼                     510
              ┌────────────────────┐
              │    Reading food    │
              │   identity data    │
              └─────────┬──────────┘
                        │
                        ▼                     515
              ┌────────────────────┐
              │  Looking up food   │
              │     identity       │
              └─────────┬──────────┘
                        │
                        ▼
       525                           520
 ┌──────────────┐   No      ◇ Is food
 │ Enter food   │◄──────────  identity listed?
 │ item identity│           ◇
 └──────┬───────┘                │ Yes
        │                        ▼
        │  535          530
        ▼        No    ◇ Is specific        540
 ┌──────────────┐◄──────  energy per unit  ┌──────────────────┐
 │Enter specific│        ◇ weight listed?   │Calculating specific│
 │energy per unit│                │ Yes     │energy of food item │
 │ weight data  │                 └─────────►└────────┬─────────┘
 └──────────────┘                                     │
                                                      ▼
                                                 ┌─────────┐
                                                 │   End   │
                                                 └─────────┘
```

# FIG. 5

**Step 810**

**Enter Recipe Mode**

Enter name of food item in the food list entry screen. If not found in Table,

Select **Recipe Mode** from Calorie Entry Options

↓

**Tare container step**

**Step 820**

Place weight of empty cooking vessel on weighing pad and Hit **Weigh Container** button. Remove container

↓

**Weigh food step**

**FIG. 6**

**Step 830**

Add ingredient i to bowl and return to weighing pad. You will be prompted for name of the ingredient which will be found (or entered) in the food database. Display shows running specific energy of recipe with each addition.

↓

**Step 840**

yes — More food items?

↓ no

Hit **Recipe Complete**.

↓

**Step 850**

Display asks if recipe will be cooked further? — yes →

**Step 860**

**Cooking adjustments**

Cook that part of the recipe.

When done cooking and cooling, place on pad and hit **Weigh cooked**. Caloric content is correct for lost preparation water.

↓ no

**Step 870**

Caloric content of the recipe is entered along with its name into the foods table

# EP 1 622 059 A1

**European Patent Office**

## EUROPEAN SEARCH REPORT

**Application Number**

EP 05 25 4765

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 2002/124017 A1 (MAULT JAMES R) 5 September 2002 (2002-09-05) * paragraph [0004] - paragraph [0007] * * paragraph [0026] - paragraph [0028] * * paragraph [0033] - paragraph [0042] * * claims 1-12 * ----- | 1-7 | G06F19/00 |
| X | US 5 233 520 A (KRETSCH ET AL) 3 August 1993 (1993-08-03) * column 7, line 60 - column 23, line 13 * * column 24, line 20 - column 25, line 43 * ----- | 1-7 | |
| X | WO 97/28738 A (ZUABE, AWNI) 14 August 1997 (1997-08-14) * page 10, line 7 - page 12, line 6 * * page 16, line 12 - line 27; figure 5 * * page 17, line 17 - line 28 * * page 21, line 3 - page 23, line 25; figure 9 * ----- | 1-7 | |
| X | GB 2 317 961 A (EDGAR DOUGLAS * GARDENER) 8 April 1998 (1998-04-08) * page 2, line 3 - page 3, line 30 * * page 4, line 14 - page 5, line 20 * ----- | 1-7 | **TECHNICAL FIELDS SEARCHED (Int.Cl.7)** <br> G06F |
| X | GB 2 269 021 A (* NEUTRON ELECTRONIC COMPUTER GES M.B.H) 26 January 1994 (1994-01-26) * page 5, line 1 - page 6, line 19 * ----- -/-- | 1-7 | |

The present search report has been drawn up for all claims

| Place of search | Date of completion of the search | Examiner |
|---|---|---|
| Munich | 25 October 2005 | Sanandrés Ledesma, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

24

**European Patent Office**

**EUROPEAN SEARCH REPORT**

Application Number

EP 05 25 4765

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | KISSIFEFF H R ET AL: "UNIVERSAL EATING MONITOR FOR CONTINUOUS RECORDING OF SOLID OR LIQUID CONSUMPTION IN MAN" AMERICAN JOURNAL OF PHYSIOLOGY, AMERICAN PHYSIOLOGICAL SOCIETY, BETHESDA, MD, US, vol. 283, 1980, pages R14-R22, XP001022526 ISSN: 0002-9513 * page R15, left-hand column, line 1 - right-hand column, line 27; figures 1,2 * * page R19, left-hand column, line 17 - line 21 * ----- | 6 | |

TECHNICAL FIELDS
SEARCHED     (Int.Cl.7)

The present search report has been drawn up for all claims

| Place of search | Date of completion of the search | Examiner |
|---|---|---|
| Munich | 25 October 2005 | Sanandrés Ledesma, J |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
 
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 05 25 4765

This annex lists the patent family members relating to the patent documents cited in the above-mentioned European search report.
The members are as contained in the European Patent Office EDP file on
The European Patent Office is in no way liable for these particulars which are merely given for the purpose of information.

25-10-2005

| Patent document cited in search report | | Publication date | Patent family member(s) | | Publication date |
|---|---|---|---|---|---|
| US 2002124017 | A1 | 05-09-2002 | AU<br>WO | 9298601 A<br>0225228 A2 | 02-04-2002<br>28-03-2002 |
| US 5233520 | A | 03-08-1993 | NONE | | |
| WO 9728738 | A | 14-08-1997 | AU<br>IL | 1455997 A<br>117064 A | 28-08-1997<br>14-11-1996 |
| GB 2317961 | A | 08-04-1998 | NONE | | |
| GB 2269021 | A | 26-01-1994 | DE<br>DE | 4204711 A1<br>4240350 A1 | 17-06-1993<br>24-06-1993 |

EPO FORM P0459

For more details about this annex : see Official Journal of the European Patent Office, No. 12/82